# DEMANDE DE BREVET EUROPEEN

(11) **EP 0 538 086 A1**
(43) Date de publication de la demande: **21.04.1993**
(21) Numéro de dépôt: 92402624.8
(22) Date de dépôt: 24.09.1992
(51) Int. Cl.: H01R 13/52, A61N 1/05

(54) **Connecteur biomédical implantable, et procédé pour réaliser une liaison électrique au moyen d'un tel connecteur**

(30) Priorité: 18.10.1991 FR 9112892
(71) Demandeur: SOURIAU ET CIE Société anonyme, 78035 Versailles (FR)
(72) Inventeur: Chincholle, Sylvie, F-92360 Meudon La Foret (FR); Herubel, Guy, F-78160 Marly le Roy (FR); Kunakey, Courage Bruno, F-78180 Montigny le Bretonneux (FR)
(74) Mandataire: Rodhain, Claude

(57) **Abrégé**

Ce connecteur comporte deux éléments homologues emboîtables (10, 20) formés chacun d'un corps isolant (12 ; 22) en matériau biocompatible auquel est fixé au moins un contact électrique, mâle ou femelle (11), venant en contact mécanique et électrique avec un contact homologue, respectivement femelle ou mâle (21), de l'élément opposé.

Selon l'invention, ces éléments homologues s'emboîtent mécaniquement avec un ajustement assurant par lui seul à la fois la solidarisation mécanique des deux éléments du connecteur et l'étanchéité des contacts vis-à-vis du milieu extérieur.

Éventuellement, les deux corps isolants emboîtés formant autour de chaque contact un volume intérieur résiduel (17, 19), ce volume est occupé par un matériau étanche de remplissage introduit après emboîtement des deux éléments du connecteur.

## Description

La présente invention concerne un connecteur à usage biomédical, et un procédé pour réaliser une liaison électrique au moyen d'un tel connecteur.

Le but premier de l'invention est de réaliser un connecteur entièrement implantable dans le corps humain ou animal, par exemple dans une région sous-cutanée, applicable à toutes les situations où l'on souhaite disposer d'une liaison électrique en principe définitive, mais éventuellement démontable, entre des faisceaux de fils (typiquement de 2 à 48 fils) intégralement implantés dans le corps d'un patient.

Un cas particulier - bien entendu nullement limitatif - est par exemple celui de la connectique utilisée par un système d'electro-stimulation pour la locomotion assistée par ordinateur, afin d'intégrer un système médical permettant par exemple la restauration de la déambulation chez un patient paraplégique.

La complexité d'un tel système d'électro-stimulation fonctionnelle implantable amène à concevoir, tant du point de vue technique que chirurgical, un système modulaire constitue de plusieurs éléments distincts interconnectés par des liaisons implantées mais éventuellement démontables, afin de permettre une intervention rapide et sélective sur ce système, et, en cas de défaillance d'un des composants, d'éviter l'extraction totale de l'implant comme cela était le cas jusqu'à présent.

Dans cet exemple, il est nécessaire de prévoir des connecteurs d'une part à proximité des électrodes de stimulation, implantées soit sur l'enveloppe du muscle soit directement sur le nerf concerné, et, d'autre part, à proximité du boîtier électronique de commande, également implanté, qui reçoit et émet des informations de commande et de contrôle par voie transcutanée vers un boîtier miniaturisé externe au corps. Le système d'interconnexion a alors pour rôle de transférer les informations issues du boîtier électronique implanté vers les terminaisons excitatrices, puis d'assurer le retour des informations résultantes vers le boîtier. Ce système doit être fiable mécaniquement et électriquement, tout en respectant la miniaturisation nécessaire à son implantation.

Cet exemple d'application illustre très bien le caractère très spécifique de la connectique biomédicale, qui impose un nombre important de contraintes résultant du caractère particulier tant de l'environnement d'utilisation (bloc opératoire) que de l'environnement fonctionnel (système implante, donc fonctionnant au sein d'un tissu vivant).

L'un des buts de la présente invention est de proposer un connecteur biomédical, qui, malgré ses très faibles dimensions, réponde à l'ensemble de ces contraintes, à savoir :
- hautes performances mécaniques, pour permettre une bonne adaptation anatomique au milieu implanté, en faisant notamment en sorte que le connecteur ne soit pas un point rigide par rapport au câble, ce dernier devant conditionner à lui seul la bonne tenue de l'ensemble,
- très grande fiabilité, les performances mécaniques (tenue des matériaux, étanchéité, etc.) et électriques (notamment la résistance de contact) devant être conservées pendant une durée de l'ordre d'une dizaine d'années,
- parfaite étanchéité vis-à-vis du milieu extérieur : en effet, le contact proprement dit doit être totalement isolé de son environnement afin de le protéger des fluides organiques qui pourraient corroder les câbles ou le connecteur par action directe, osmose, etc., et également contre la pénétration intérieure des cellules qui se forment au cours de la phase de réhabitation des tissus dans la zone d'implantation ; d'autre part, les fluides biologiques étant des électrolytes, il est indispensable d'éviter à tout prix leur infiltration dans le système, une telle infiltration entraînant des complications électriques et une fuite de courant dans le corps pouvant détruire de façon irreversible les tissus avoisinants,
- biocompatibilité totale, afin d'éviter toute réaction tissulaire de rejet et permettre, au contraire, la «réhabitation» tissulaire de la zone implantée, c'est-à-dire la croissance de cellules autour des éléments implantés (connecteurs, fils, électrodes, etc. ),
- Résistance aux conditions thermiques et chimiques imposées par la stérilisation du matériel, qu'il s'agisse de traitements à la vapeur (températures de l'ordre de 200°C) et/ou de traitements à froid par trempage dans des liquides de stérilisation spéciaux.

L'une des difficultés de la réalisation d'un tel connecteur tient au fait que la taille et le poids réduits excluent notamment tout recours aux moyens classiques d'étanchéité tels que joint ou presse-étoupe, qui ne peuvent être envisagés en aucune façon dans cette application de connecteur miniature à usage biomédical.

A cet effet, le connecteur biomédical de l'invention, qui comporte deux éléments homologues emboîtables formes chacun d'un corps isolant en matériau biocompatible auquel est fixé au moins un contact électrique, mâle ou femelle, venant en contact mécanique et électrique avec un contact homologue, respectivement femelle ou mâle, de l'élément oppose, est caractérisé en ce que ces éléments homologues s'emboîtent mécaniquement avec un ajustement assurant par lui seul à la fois la solidarisation mécanique des deux éléments du connecteur et l'étanchéité des contacts vis-à-vis du milieu extérieur ; éventuellement, les deux corps isolants emboîtés formant autour de chaque contact un volume intérieur résiduel, ce volume est occupe par un matériau étanche de remplissage introduit après emboîtement des deux éléments du connecteur.

Le procédé mettant en oeuvre un tel connecteur, qui est destiné à réaliser une liaison électrique démontable entre au moins deux fils homologues, est caractérisé par les étapes de : soudure de chaque fil à son contact correspondant; placement et solidarisation mécanique des contacts pourvus de leurs fils au corps isolant de leur élément de connecteur correspondant ; emboîtage des deux éléments du connecteur; introduction éventuelle d'un matériau étanche de remplissage dans le volume intérieur résiduel forme autour de chaque contact ; et enrobage éventuel de l'ensemble ainsi constitue par une gaine de matériau isolant biocompatible.

On va maintenant décrire un certain nombre d'exemples de réalisation du connecteur selon l'invention, en référence aux dessins annexes.

La figure 1 est une vue éclatée, en coupe longitudinale, d'un exemple de connecteur monocontact selon l'invention.

La figure 2 montre, à échelle agrandie, le connecteur de la figure 1 une fois assemblé et implanté.

La figure 3 illustre un exemple de réalisation de connecteur hermaphrodite à deux contacts.

La figure 4 illustre un exemple de connecteur plat à 24 contacts réalisé conformément au même concept.

Les figures 5 et 6 montrent deux manières possibles d'utiliser le connecteur multicontact de la figure 4.

La figure 7 est une vue en coupe d'un exemple de connecteur rond à 9 contacts réalisé selon le même concept que celui des figures 1 et 2.

La figure 8 est une vue en plan de l'élément inférieur du connecteur de la figure 7.

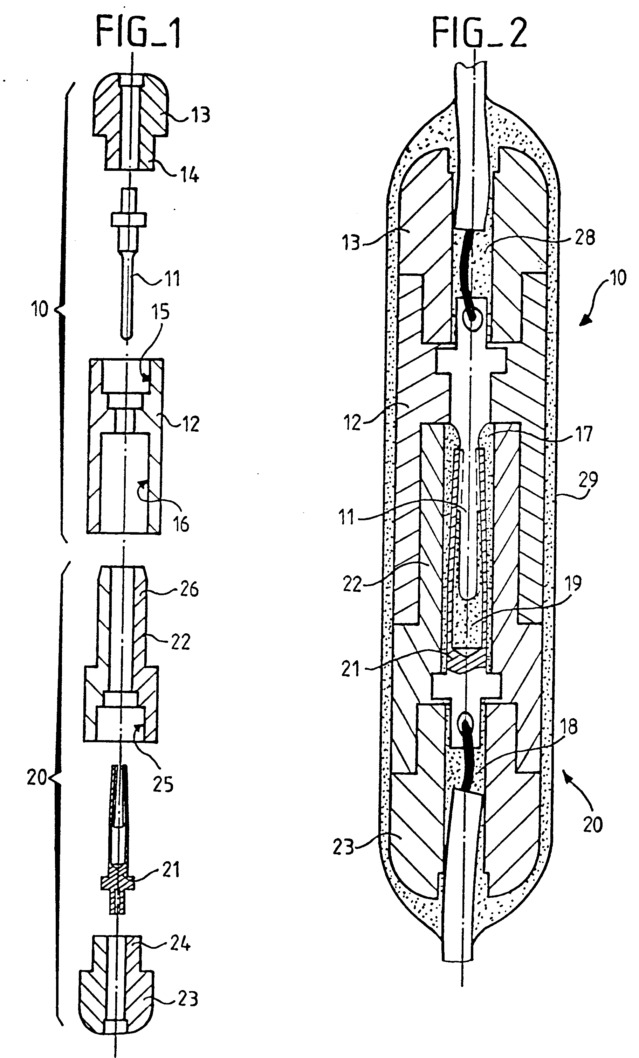

Sur la figure 1, on a illustré le connecteur de l'invention dans une configuration monocontact; on verra par la suite que l'on peut réaliser aisément des connecteurs multicontact sur le même principe de base.

Le connecteur comporte essentiellement deux éléments homologues de connecteur 10 et 20 comportant chacun, essentiellement, un contact électrique métallique 11, 21 inséré dans un corps isolant 12, 22 fermé en partie arrière par un capuchon 13, 23. Dans la configuration illustrée, le corps isolant 12 correspond à un élément femelle, et reçoit un contact électrique mâle, tandis que le corps isolant 22 correspond à un élément mâle et reçoit un contact électrique femelle, ce choix n'étant bien entendu pas limitatif.

En ce qui concerne les matériaux, les corps isolants 12, 22 et les capuchons 13, 23 sont réalisés de préférence en PTFE, PMMA ou PSU, ou en en élastomère silicone éventuellement arme, qui sont des matériaux biocompatibles parfaitement adaptés à une application biomédicale. Les contacts métalliques 11, 21 sont réalisés en un métal ou alliage approprié tel que le platine et ses dérivés iridiés, le titane et ses alliages, les alliages de cobalt, ou des aciers inoxydables tels que la nuance 316L VM (*Vacuum Molted* : fondu sous vide), le choix étant déterminé de manière à assurer un bon compromis entre la souplesse de l'alliage, la bonne tenue mécanique et une bonne résistivité.

De façon caractéristique de l'invention, l'étanchéité et la solidarisation mécanique des deux éléments de connecteur entre eux, comme celle des capuchons sur les corps isolants, sont assurées par un ajustement étroit des surfaces, à savoir l'ajustement entre l'alésage 16 de la partie femelle du corps 12 et le diamètre extérieur de la partie cylindrique mâle 26 de l'élément 22. On verra en outre que cet ajustement permet à la partie mâle 26 d'agir en tant que piston à l'intérieur de la cavité 16, permettant de chasser tout fluide organique qui pourrait se trouver à l'intérieur de cette cavité lors de l'accouplement des connecteurs. En ce qui concerne les capuchons 13 et 23, on a de même un ajustement étroit entre les parties cylindriques 14, 24 et les logements homologues 15, 25 des corps 12 et 22.

En ce qui concerne la forme extérieure, le connecteur une fois assemblé a une forme parfaitement cylindrique avec des bouts arrondis, forme optimisée pour éviter des réactions tissulaires de rejet.

On va maintenant décrire la manière dont sont assembles les éléments de la figure 1 pour aboutir à la structure finale de la figure 2, telle qu'implantée.

Tout d'abord, on soude à chacun des contacts 11 et 21 le fil correspondant. Compte tenu de l'environnement spécifique, la soudure utilisée est du type soudure laser ou soudure électrique par points, afin d'assurer une liaison fiable et résistante. Le câble se présente sous forme d'un cylindre de 1 mm de diamètre, et présente une élasticité importante (de l'ordre de 12%), nécessaire pour tenir compte des mouvements des membres du sujet recevant l'installation implantée. Ce câble a bien entendu toutes les caractéristiques mécaniques et élastiques spécifiques à l'usage biomédical envisagé.

Les contacts pourvus de leurs fils sont alors mis en place dans le corps respectif 12, 22 et solidarisés à celui-ci, par exemple par mise en place des capuchons 13, 23.

La connexion est alors prête à être réalisée par réunion des deux éléments de connecteurs. Au cours de cette opération, l'ajustement entre les surfaces homologues 16 et 26 fait en sorte que tout fluide organique qui pourrait se trouver dans l'espace compris entre les deux éléments de connecteur est chasse vers l'extérieur via l'interstice entre les pièces coopérantes, du fait de la souplesse de celles-ci.

On soulignera ici l'importance de cette caractéristique, rendue nécessaire par le fait que la connexion est le plus souvent réalisée *in situ*, conditions pour lesquelles le connecteur de la présente invention est conçu et auxquelles il est parfaitement adapté.

Une fois cette opération réalisée, on peut injecter de chaque côté du connecteur, par les trous de passage des fils dans les capuchons, une colle médicale (par exemple une colle silicone) qui va venir remplir le volume intérieur subsistant autour des deux éléments métalliques en contact, aussi bien à l'extérieur de ceux-ci (région repérée 17 sur la figure) qu'entre ceux-ci (région repérée 19 sur la figure). En poursuivant cette injection, on remplit également les orifices 18, 28 de passage des fils dans les capuchons, de sorte que le connecteur ne comprend alors plus aucun volume résiduel interne.

De même, on peut également enrober l'ensemble du connecteur muni de ses terminaisons de fils dans une gaine 29, par exemple en un matériau silicone surmoule, avant d'implanter le tout.

L'ensemble ainsi forme peut avoir des dimensions aussi faibles qu'un diamètre de 3 mm pour une longueur de 17 mm et une masse de 0,54 g.

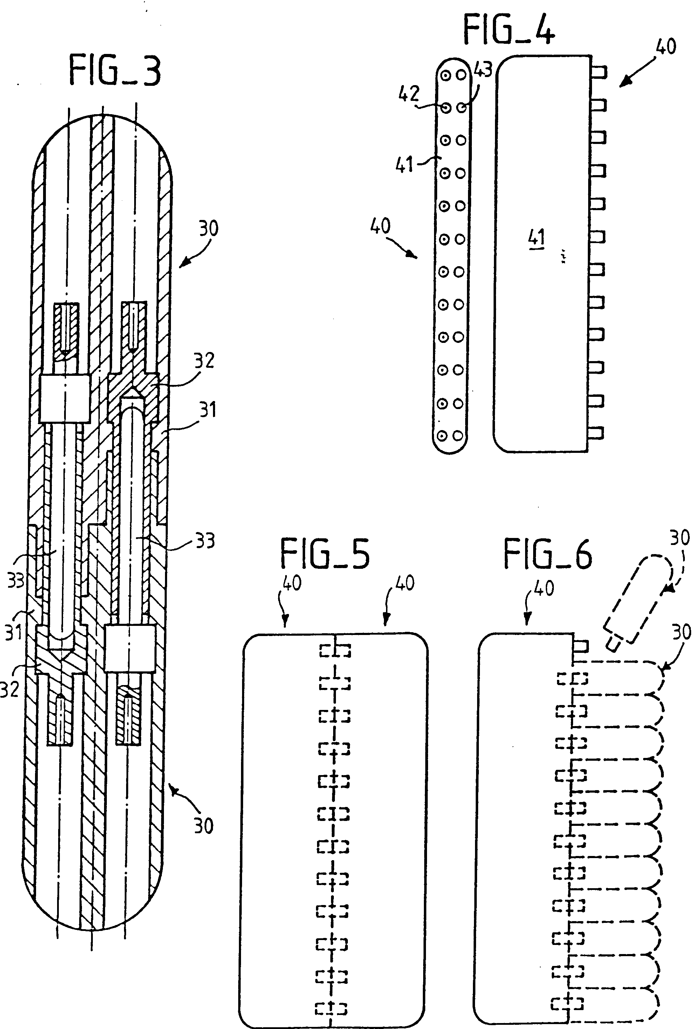

Sur les figures 3 à 8, on a représenté des applications du même concept à des connecteurs à plusieurs contacts.

Ainsi, sur la figure 3 on a illustré un connecteur hermaphrodite destiné à relier des paires de fils. Les deux éléments de connecteur 30 sont en tout point identiques et comportent chacun corps isolant 31 recevant un contact femelle 32 et un contact mâle 33 placés côte à côte.

Le processus d'accouplement des deux éléments de ce connecteur est similaire à celui du connecteur monocontact de la figure 1. On notera en outre que sa forme hermaphrodite assure un détrompage immédiat à l'accouplement entre les deux fils de la paire.

Sur la figure 4, on a illustré un connecteur à 24 contacts réalisé selon les mêmes enseignements.

L'élément de connecteur 40 comporte ainsi un corps isolant 41 portant une série de contacts femelles 42 et une série de contacts mâles 43, disposés suivant deux rangées parallèles. Le connecteur peut par exemple relie à une nappe de fils provenant d'un boîtier de commande.

Les figures 5 et 6 illustrent deux manières d'utiliser cet élément de connecteur: sur la figure 5, on fait coopérer ensemble deux connecteurs 40 identiques à 24 contacts, par exemple pour relier entre elles deux nappes de fils, tandis que sur la figure 6 on connecte à chaque paire de contacts mâle/femelle 42/43 du connecteur 40 un connecteur bicontact tel que celui illustré à la figure 3, les divers connecteurs 30 étant plaqués les uns contre les autres et l'ensemble pouvant être si nécessaire finalement enrobé dans une gaine de matériau silicone ; cette dernière configuration peut être notamment utilisée lorsqu'il s'agit de distribuer vers une pluralité d'électrodes placées en différents points du corps les signaux d'excitation achemines sur une nappe en provenance d'un boîtier de commande.

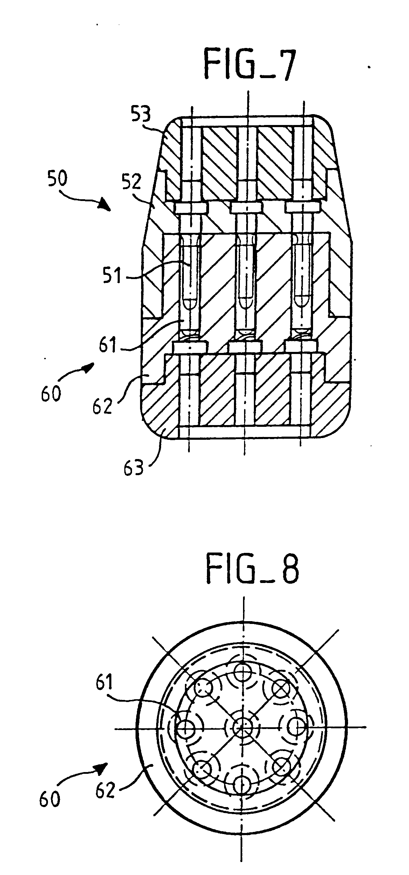

Les figures 7 et 8 illustrent encore une autre forme possible de réalisation d'un connecteur selon l'invention.

Le connecteur illustré sur ces figures est un connecteur à 9 contacts (ou 8 contacts + 1 masse) répartis selon une configuration circulaire symétrique. Le connecteur comporte deux éléments 50, 60 portant, l'un, des contacts mâles 51 et, l'autre, des contacts femelles 61, ces contacts étant disposés dans un corps isolant 52, 62 fermé en partie arrière par un capuchon 53, 63.

On peut éventuellement, en fonction des contraintes locales d'implantation, donner au connecteur une forme non symétrique par rapport au plan médian, avec par exemple un élément 50 terminé par une partie arrière «fuyante» et l'élément 60 terminé par une partie arrière sensiblement plane.

Bien entendu, l'invention n'est pas limitée aux formes de réalisation décrites ici. En particulier, tous les types de connecteur décrits (monocontact ou multicontact) peuvent être réalisés en une technologie surmoulée, le contact préalablement muni de son fil constituant un insert autour duquel on forme en une seule opération le corps par injection d'une résine, donc sans assemblage mécanique de pièces distinctes.

## Revendications

1. Un connecteur biomédical, comportant deux éléments homologues emboîtables (10, 20; 30, 30; 40, 30; 40, 40) formés chacun d'un corps isolant (12; 22; 31 ; 41) en matériau biocompatible auquel est fixé au moins un contact électrique, mâle ou femelle (11 ; 33 ; 43), venant en contact mécanique et électrique avec un contact homologue, respectivement femelle ou mâle (21 ; 32 ; 42), de l'élément opposé, caractérisé en ce que ces éléments homologues s'emboîtent mécaniquement avec un ajustement assurant par lui seul à la fois la solidarisation mécanique des deux éléments du connecteur et l'étanchéité des contacts vis-à-vis du milieu extérieur.

2. Le connecteur biomédical de la revendication 1 dans lequel, les deux corps isolants emboîtés formant autour de chaque contact un volume intérieur résiduel (17, 19), ce volume est occupé par un matériau étanche de remplissage introduit après emboîtement des deux éléments du connecteur.

3. Un procédé pour réaliser une liaison électrique démontable entre au moins deux fils homologues au moyen d'un connecteur biomédical comportant deux éléments homologues emboîtables formés chacun d'un corps isolant auquel est fixé au moins un contact électrique, mâle ou femelle, venant en contact mécanique et électrique avec un contact homologue, respectivement femelle ou mâle, de l'élément opposé, caractérisé par les étapes suivantes :
- soudure de chaque fil à son contact correspondant,
- placement et solidarisation mécanique des contacts pourvus de leurs fils au corps isolant de leur élément de connecteur correspondant, et
- emboîtage des deux éléments du connecteur.

4. Le procédé de la revendication 3, comprenant en outre, après emboîtage des deux éléments du connecteur, une étape d'introduction d'un matériau étanche de remplissage dans le volume intérieur résiduel formé autour de chaque contact.

5. Le procédé de la revendication 3, comprenant en outre une étape finale d'enrobage de l'ensemble par une gaine de matériau isolant biocompatible.
